# EUROPEAN PATENT SPECIFICATION

(11) **EP 2 938 298 B1**
(45) Date of publication and mention of the grant of the patent: **02.08.2017**
(21) Application number: 13824497.5
(22) Date of filing: 24.12.2013
(51) Int. Cl.: A61F 2/66

(54) **ARTIFICIAL FOOT**
FUSSPROTHESE
PIED ARTIFICIEL

(30) Priority: 27.12.2012 IT VR20120251
(43) Date of publication of application: 04.11.2015
(73) Proprietor: Hornos, Pedro, 25123 Brescia (IT); Tagliafierro, Mario, 25123 Brescia (IT)
(72) Inventor: Hornos, Pedro, 25123 Brescia (IT); Tagliafierro, Mario, 25123 Brescia (IT)
(74) Representative: Modiano, Micaela Nadia
(86) International application number: PCT/EP2013/077974
(87) International publication number: WO 2014/102269

(56) References cited:
- WO-A2-2011/066354
- US-A- 5 258 039
- US-A- 6 071 313
- US-A1- 2006 212 131

## Description

The present invention relates to an artificial foot.

Nowadays several different types of artificial foot are known and are widely used.

Specifically, a first type makes use of plate-like elements which extend along a direction of longitudinal extension: the plate-like element defines, at the front and at the rear, a respective resting portion.

Typically at the rear region, the plate-like element is connected, by way of connection means, to the body of the user.

In a first generation of the artificial foot described above, the plate-like element is coupled to a body, which is also plate-like, at the rear of the artificial foot.

In a second generation the plate-like element is associated with an artificial ankle, for example motorized, which is designed to enable a movement of the artificial foot that is closer to the natural movement, during the impact with the ground.

Such solution, which is evidently valid from a conceptual point of view, is however extremely complex and costly in terms both of its production and of its maintenance.

US2006/212131 A1 describes a prosthetic foot comprising a rear foot portion having an attachment platform and a heel strike, and a front foot portion having a momentum interrupter and a toe plate. As the foot approaches toe-off, the toe plate flexibly deflects upwards and the momentum interrupter compresses.

US6,071,313 A1 describes an artificial foot in accordance with the preamble of claim 1. The aim of the present invention is to provide an artificial foot that is provided with a simple structure but is capable of ensuring extremely high levels of performance under all conditions of use.

Within this aim, an object of the present invention is to provide an artificial foot that is versatile and modulable as a function of the requirements of of the user.

Another object of the invention is to devise an artificial foot that is low cost, so as to make its use advantageous from an economic viewpoint as well.

This aim and these and other objects which will become better apparent hereinafter are all achieved by an artificial foot according to claim 1.

Further characteristics and advantages of the invention will become better apparent from the description of some preferred, but not exclusive, embodiments of an artificial foot according to the present invention, which are illustrated by way of non-limiting example in the accompanying drawings wherein:
Figure 1 is a perspective view of a first embodiment of an artificial foot according to the invention;
Figure 2 is an exploded view of the first embodiment of the artificial foot;
Figure 3 is a view from above of the first embodiment of the artificial foot according to the invention;
Figure 4 is a cross-sectional view taken along the line IV-IV in Figure 3;
Figure 5 is a perspective view of a second embodiment of an artificial foot according to the invention;
Figure 5a is another perspective view of the second embodiment of the artificial foot, connected to an elongated element for connection to the body of the user;
Figure 6 is an exploded view of the second embodiment of the artificial foot;
Figure 7 is a view from above of the second embodiment of the artificial foot according to the invention;
Figure 8 is a cross-sectional view taken along the line VIII-VIII in Figure 7;
Figure 9 is a perspective view of a third embodiment of an artificial foot according to the invention;
Figure 10 is an exploded view of the third embodiment of the artificial foot;
Figure 11 is a view from above of the third embodiment of the artificial foot according to the invention;
Figure 12 is a cross-sectional view taken along the line XII-XII in Figure 11;

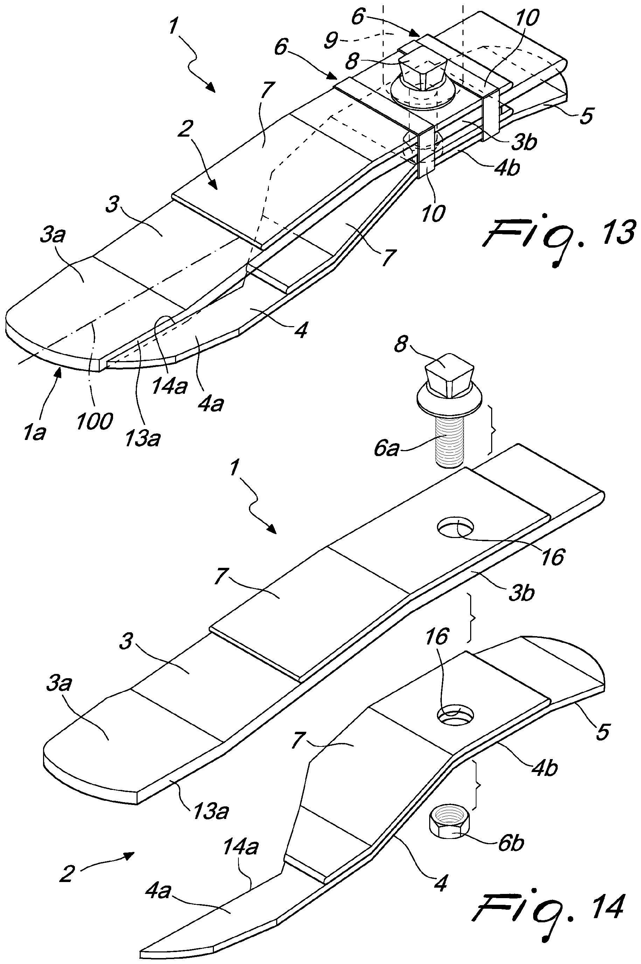

Figure 13 is a perspective view of a fourth embodiment of an artificial foot according to the invention;
Figure 14 is an exploded view of the fourth embodiment of the artificial foot;
Figure 15 is a view from above of the fourth embodiment of the artificial foot according to the invention;
Figure 16 is a cross-sectional view taken along the line XV-XV in Figure 15;
Figure 17 is a cross-section similar to the one in Figure 16 of a further variation of embodiment.

In the embodiments illustrated, individual characteristics shown in relation to specific examples may in reality be interchanged with other, different characteristics, existing in other embodiments.

With reference to the figures, an artificial foot, which is generally indicated with the reference numeral 1, comprises a resting element 2 that is to rest on the ground and is extended along a longitudinal extension axis 100.

The resting element 2 is associated with an engagement body 8 for its connection to the body of the user.

In this regard, it is possible to connect the conventional engagement body 8, such as a plate-like element or a coupler, to an elongated element 9 which is in turn associated with or can be associated with the body of the user.

According to the present invention, the resting element 2 defines a rear resting portion 5 and two front resting portions 3a and 4a that are mutually spaced transversely to the longitudinal extension axis 100.

Specifically, the two front resting portions 3a and 4a are adapted to be affected by forces, during use, independently of each other. The resting element 2 comprises a first resting body 3 and a second resting body 4 that have an elongated extension.

The first resting body 3 defines, at a front region, the front resting portion 3a and the second resting body 4 defines, at a front region, the front resting portion 4a.

Specifically, the first resting body 3 and the second resting body 4 have a divergent extension, with respect to the longitudinal extension axis 100, going from the rear resting portion 5 toward the front resting portions 3a and 4a.

Consequently, the respective front regions of the first resting body 3 and of the second resting body 4 are arranged mutually side-by-side along a direction that is substantially transverse to the longitudinal extension axis 100 of the elongated element 2. The mutually facing longitudinal edges 13a and 14a of the front portions 3a and 4a are arranged mutually spaced apart, indicatively by approximately 1.5 - 3 mm, so as to avoid fretting against each other during use. At least one portion of the rear region 3b of the first resting body 3 is arranged so as to face at least one portion of the rear region 4b of the second resting body 4.

In particular, the rear region of the first resting body 3 is arranged above the rear region of the second resting body 4.

Advantageously, the front region of the first resting body 3 has a longer extension in a transverse direction than the front region of the second resting body 4.

Specifically, the front region of the first resting body 3 is directed toward the inside of the artificial foot 1 and the front region of the second resting body 4 is directed toward the outside of the artificial foot 1.

The front portion 3a of the first resting body 3 is arranged closer to the toe 1a of the artificial foot 1 with respect to the front portion 4a of the second resting body 4.

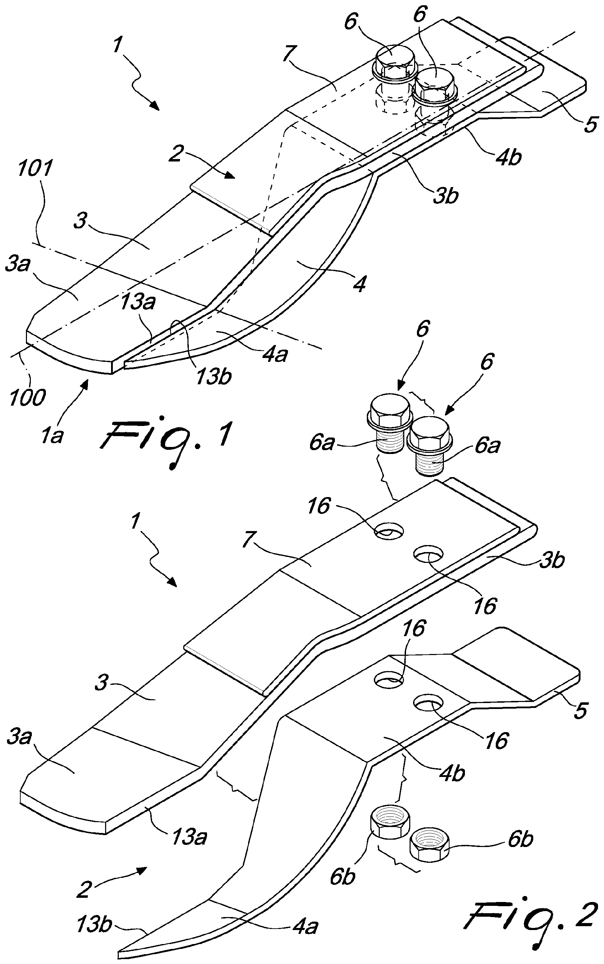

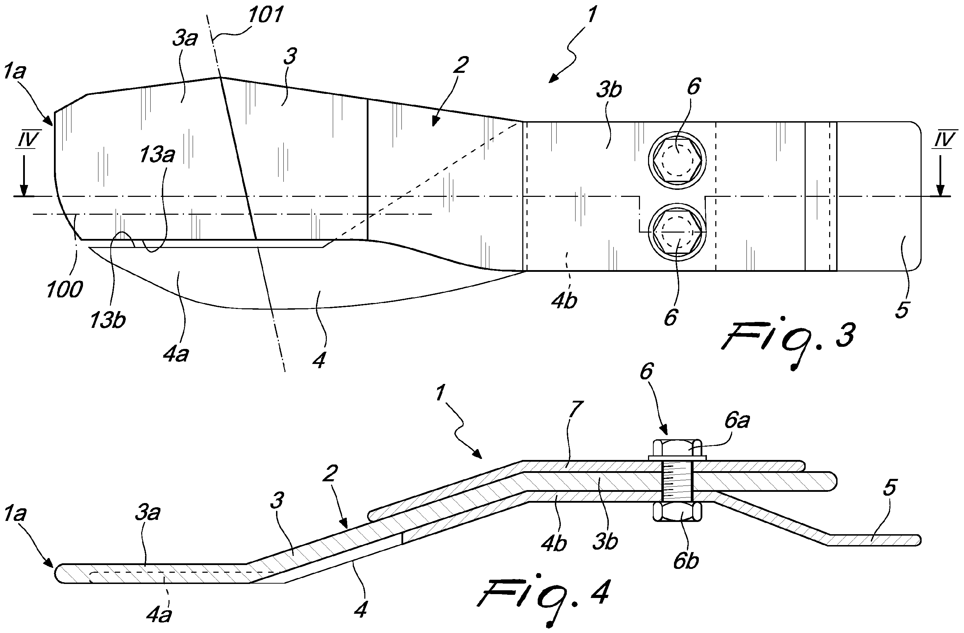

With reference to the embodiment shown in Figures 1 to 4, it is likewise possible that the rear edges of the front portions 3a and 3b do not extend at right angles to the longitudinal extension axis 100 but along an inclined direction 101 that approaches the toe going from the outside toward the inside of the artificial foot 101.

According to the present invention, the artificial foot 1 comprises means for mutual locking 6 between the first resting body 3 and the second resting body 4 that are arranged substantially at the respective second rear regions.

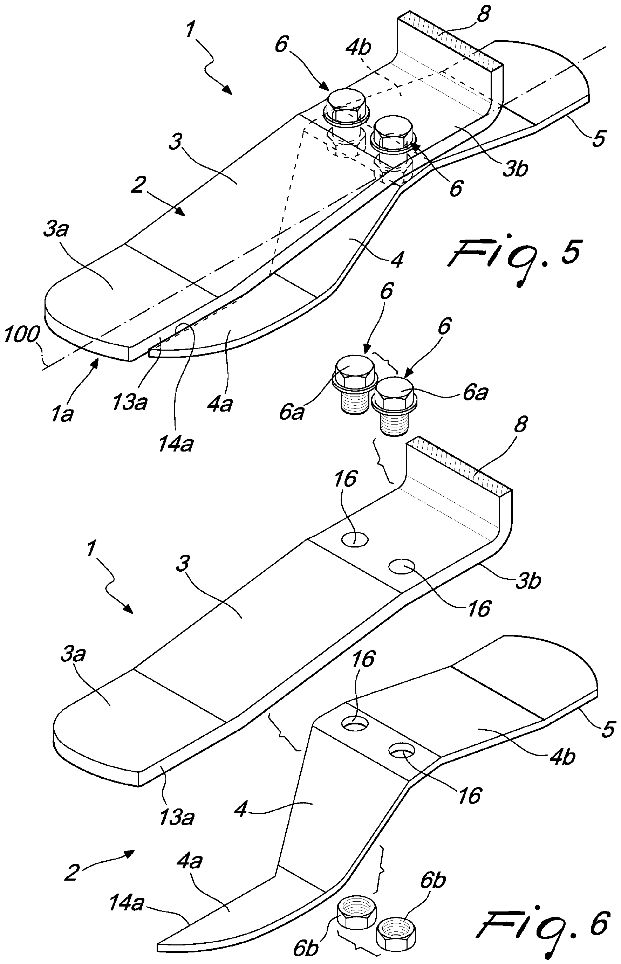

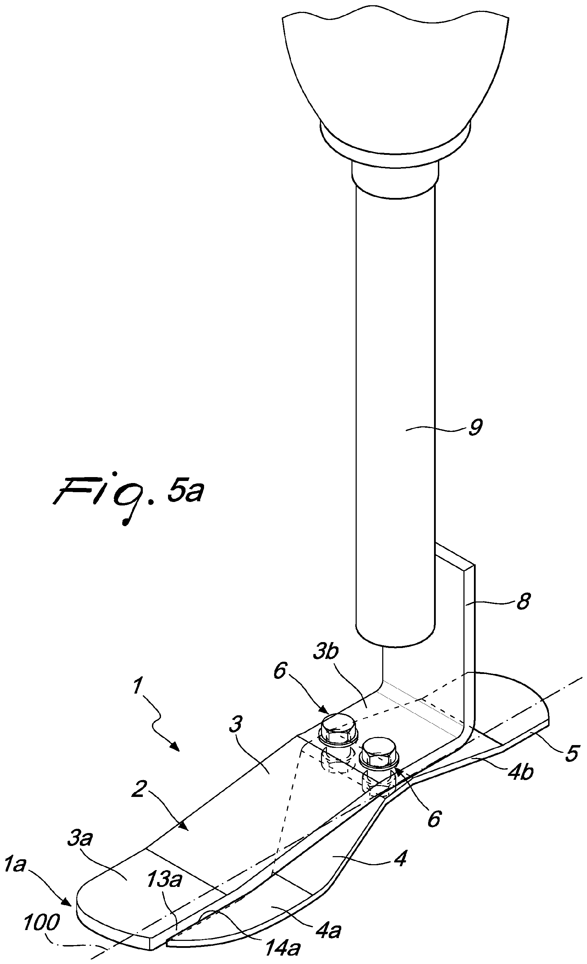

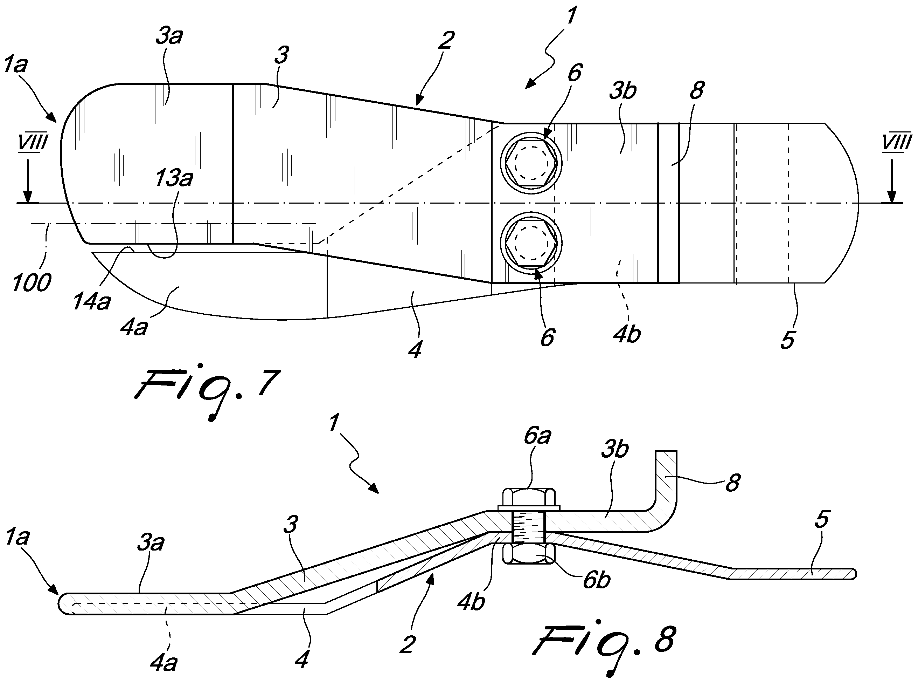

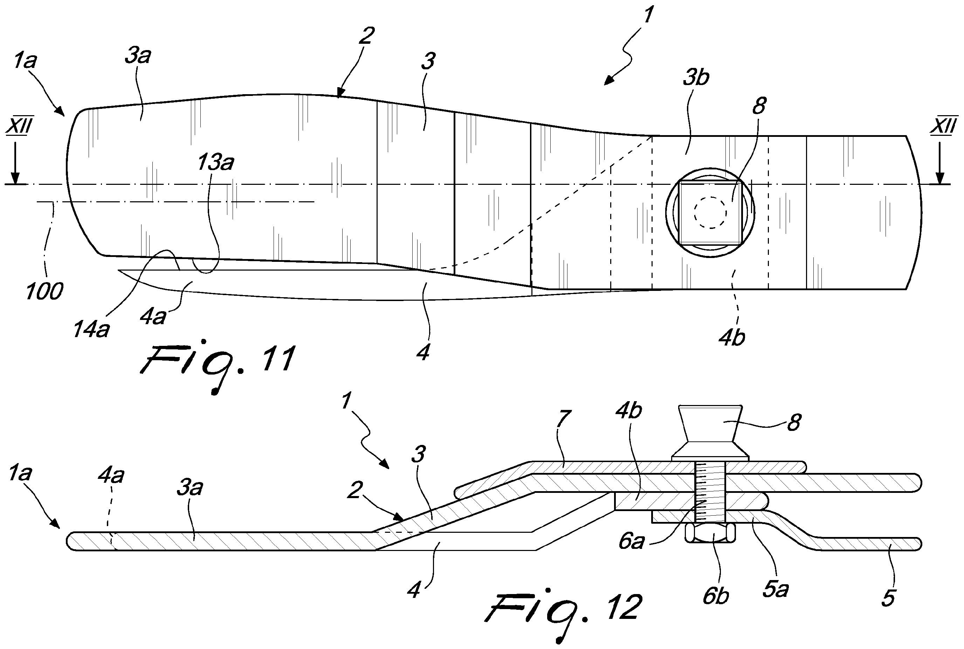

With reference to the embodiments shown in Figures 1 to 12, the mutual locking means 6 can be constituted by one or more threaded elements 6a, which are insertable into through openings 16 which are provided at the rear region 3b and 4b of the first resting body 3 and of the second resting body 4, and by respective locking nuts 6b which are screwed onto the free ends of the threaded elements 6a.

Preferably, the locking means 6 can be constituted by two threaded elements 6a, which can be associated with two respective locking nuts 6b, and arranged mutually spaced, as illustrated, transversely or longitudinally: in this regard it has been found that arranging the two threaded elements 6a longitudinally can ensure a greater lever arm arm and, thus, a greater rigidity in the connection between the first resting body 3 and the second resting body 4.

Naturally, there is no reason why different mutual locking means 6 cannot be used, such as, for the purposes of example, bands 10 that extend transversely with respect to the direction of longitudinal extension 100.

Conveniently, the second resting body 4 defines, at its rear end, the rear resting portion 5.

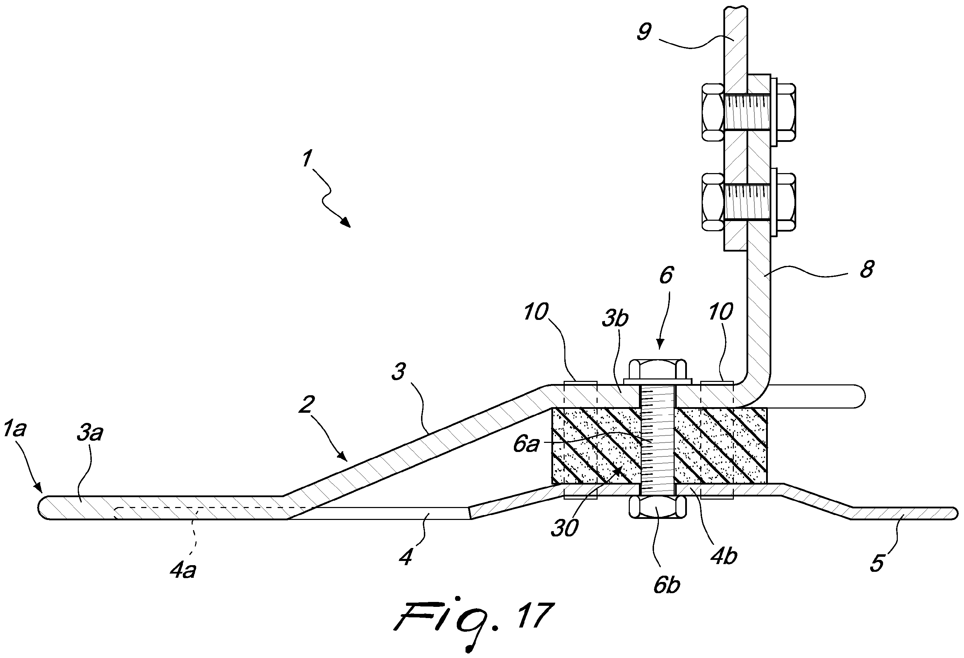

As shown in Figure 17, it is possible for the second resting body 4 to lie, for the most part, substantially on the same plane of arrangement.

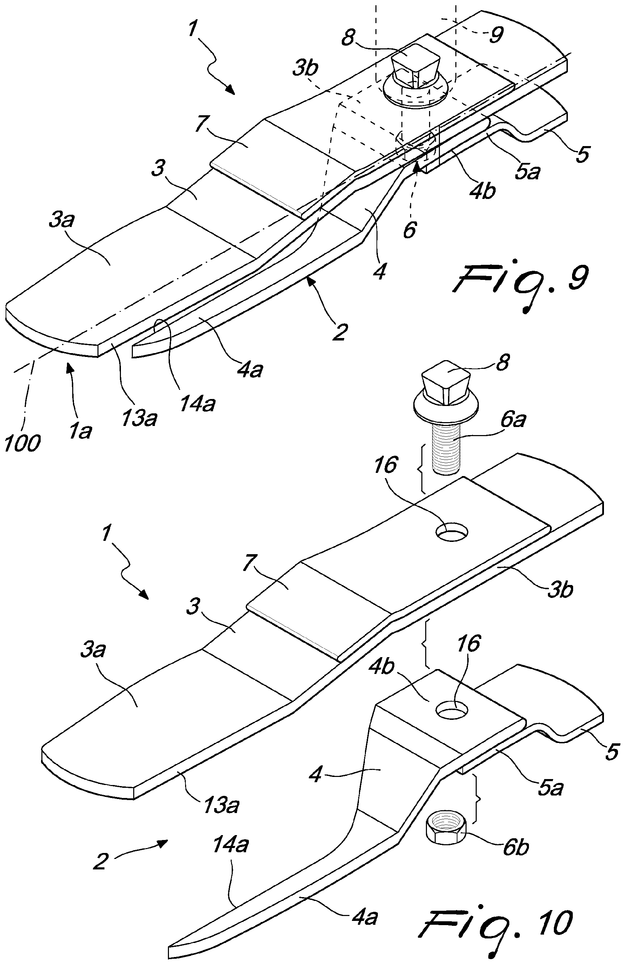

As shown in Figures 10 to 12, there is no reason why there cannot be a rear resting body 5a that defines the rear resting portion 5 and which can be associated in a downward region with the second resting body 4.

The rear regions 3b and 4b of the first and of the second resting body 3 and 4 are, advantageously, arranged on a plane of arrangement that is raised with respect to the plane of arrangement of the front regions.

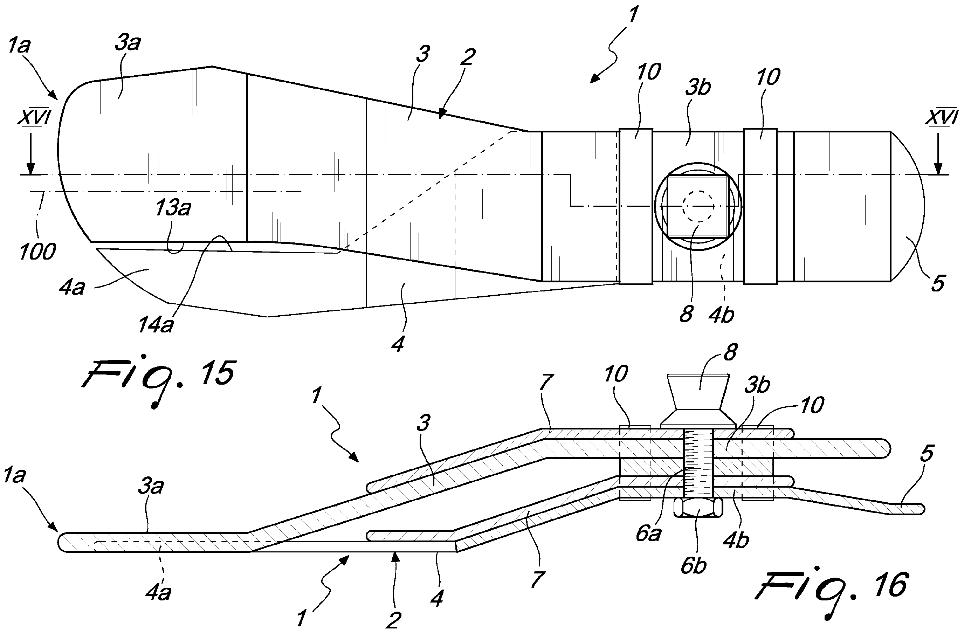

As illustrated in Figure 16, it is possible that the rear region 4b of the second resting body 4 can rest on a plane of arrangement that, in use, is close to the ground.

With reference to such embodiment, in order to maintain the rear region 3b of the first resting body 3 in the raised position and distanced from the rear region 4b, it is possible to provide a spacing and cushioning body 30.

Such spacing and cushioning body 30 can be constituted by a parallelepiped element made of elastic material, or by a series of Belleville springs, and is arranged between the mutually facing surfaces of the two rear regions 3b and 4b.

If it is desired to further modulate the behavior under compression and, consequently, the stress-response behavior of the first and of the second resting body 3 and 4, it is possible for the spacing and cushioning body 30 to have areas of different elastic resistance, for example, along the direction of longitudinal extension 100.

In order to allow for adaptation of the stress-response characteristics of the artificial foot 1, there can be at least one plate-like stiffening body 7 arranged so as to face a respective connection region that is defined on the first resting body 3 and/or on the second resting body 4.

As indicated, the presence of the plate-like stiffening body or bodies 7 is optional.

The use of the artificial foot 1 according to the present invention is evident from the foregoing description.

Specifically, it has been found that, thanks to the innovative structure of the resting element 2 constituted by the first resting body 3 and by the second resting body 4, it is possible to stress, during use, independently, two front portions 3a and 4a which are arranged transversely spaced apart.

In this manner, the behavior of the artificial foot 1 during impact with the ground comes to substantially correspond to the behavior of the foot.

All the characteristics of the invention, indicated above as advantageous, convenient or similar, may also be missing or be substituted by equivalent characteristics.

The invention, thus conceived, is susceptible of numerous modifications and variations, all of which are within the scope of the appended claims.

In practice it has been found that in all the embodiments the invention has achieved the intended aim and objects.

In practice the materials employed, as well as the dimensions and the contingent shapes, may be any according to requirements.

Moreover, all the details may be substituted by other, technically equivalent elements.

Where the technical features mentioned in any claim are followed by reference numerals and/or signs, those reference numerals and/or signs have been included for the sole purpose of increasing the intelligibility of the claims and accordingly, such reference numerals and/or signs do not have any limiting effect on the interpretation of each element identified by way of example by such reference numerals and/or signs.

## Claims

1. An artificial foot (1), comprising a resting element (2) that is to rest on the ground and is extended along a longitudinal extension axis (100), said resting element (2) being associated with an engagement body (8) for its connection to the body of the user, said resting element (2) defining a rear resting portion (5) and two front resting portions (3a, 4a) that are mutually spaced transversely to the longitudinal extension axis (100) and are adapted to be affected by forces, during use, independently of each other,
said resting element (2) comprises a first resting body (3) and a second resting body (4) that have an elongated extension, **characterized in that** said first resting body (3) and said second resting body (4) having a divergent extension with respect to the longitudinal extension axis (100), going from the rear resting portion (5) toward the front resting portions (3a, 4a), said first resting body (3) and said second resting body (4) defining respectively a rear region and a front region, at least one portion (3b) of the rear region of said first resting body (3) being arranged so as to face at least one portion (4b) of the rear region of said second resting body (4).

2. The artificial foot (1) according to claim 1, **characterized in that** the respective front regions of said first resting body (3) and said second resting body (4) are arranged mutually side-by-side along a direction that is substantially transverse to the longitudinal extension axis (100) of said elongated element (2).

3. The artificial foot (1) according to one or more of the preceding claims, **characterized in that** the rear region of said first resting body (3) is arranged above the rear region of said second resting body (4).

4. The artificial foot (1) according to one or more of the preceding claims, **characterized in that** the front region of said first resting body (3) has a longer extension in a transverse direction than the front region of said second resting body (4).

5. The artificial foot (1) according to one or more of the preceding claims, **characterized in that** the front region of said first resting body (3) is directed toward the inside of said artificial foot (1) and **in that** the front region of said second resting body (4) is directed toward the outside of said artificial foot (1).

6. The artificial foot (1) according to one or more of the preceding claims, **characterized in that** the front portion (3a) of said first resting body (3) is arranged closer to the toe (1a) of said artificial foot (1) than the front portion (4a) of said second resting body (4).

7. The artificial foot (1) according to one or more of the preceding claims, **characterized in that** it comprises means (6) for mutual locking between said first resting body (3) and said second resting body (4) that are arranged substantially at the respective second rear regions.

8. The artificial foot (1) according to one or more of the preceding claims, **characterized in that** said second resting body (4) defines said rear resting portion (5).

9. The artificial foot (1) according to one or more of the preceding claims, **characterized in that** said rear regions are arranged on a plane of arrangement that is raised with respect to the plane of arrangement of said front regions.

10. The artificial foot (1) according to one or more of the preceding claims, **characterized in that** it comprises at least one plate-like stiffening body (7) that is arranged so as to face a respective connection region defined on said first resting body (3) and/or on said second resting body (4).

## Patentansprüche

1. Eine Fußprothese (1), die ein Auflageelement (2) umfasst, das auf dem Boden aufliegen soll und sich entlang einer Längserstreckungsachse (100) erstreckt, wobei das Auflageelement (2) mit einem Eingriffskörper (8) zum Zwecke seiner Verbindung mit dem Körper des Benutzers verbunden ist, wobei das Auflageelement (2) einen hinteren Auflageabschnitt (5) und zwei vordere Auflageabschnitte (3a, 4a) bestimmt, die quer zur Längserstreckungsachse (100) voneinander beabstandet und ausgebildet sind, um im Gebrauch unabhängig voneinander von Kräften beeinflusst zu werden,
das Auflageelement (2) umfasst einen ersten Auflagekörper (3) und einen zweiten Auflagekörper (4), die eine verlängerte Ausdehnung haben, **dadurch gekennzeichnet, dass**
der erste Auflagekörper (3) und der zweite Auflagekörper (4) eine sich verzweigende Ausdehnung mit Bezug auf die Längserstreckungsachse (100) haben, verlaufend vom hinteren Auflageabschnitt (5) zu den vorderen Auflageabschnitten (3a, 4a), wobei der erste Auflagekörper (3) und der zweite Auflagekörper (4) einen hinteren Bereich beziehungsweise einen vorderen Bereich bestimmen, mindestens ein Abschnitt (3b) des hinteren Bereichs des ersten Auflagekörpers (3) so angeordnet ist, dass er mindestens einem Abschnitt (4b) des hinteren Bereichs des zweiten Auflagekörpers (4) zugewandt ist.

2. Die Fußprothese (1) gemäß Anspruch 1, **dadurch gekennzeichnet, dass** die jeweiligen vorderen Bereiche des ersten Auflagekörpers (3) und des zweiten Auflagekörpers (4) entlang einer Richtung, die im Wesentlichen quer zur Längserstreckungsachse (100) des verlängerten Elements (2) ist, nebeneinander angeordnet sind.

3. Die Fußprothese (1) gemäß einem oder mehreren der obigen Ansprüche, **dadurch gekennzeichnet, dass** der hintere Bereich des ersten Auflagekörpers (3) oberhalb des hinteren Bereichs des zweiten Auflagekörpers (4) angeordnet ist.

4. Die Fußprothese (1) gemäß einem oder mehreren der obigen Ansprüche, **dadurch gekennzeichnet, dass** der vordere Bereich des ersten Auflagekörpers (3) eine längere Ausdehnung in eine transversale Richtung hat als der vordere Bereich des zweiten Auflagekörpers (4).

5. Die Fußprothese (1) gemäß einem oder mehreren der obigen Ansprüche, **dadurch gekennzeichnet, dass** der vordere Bereich des ersten Auflagekörpers (3) zur Innenseite der Fußprothese (1) hin gerichtet ist, und dadurch, dass der vordere Bereich des zweiten Auflagekörpers (4) zur Außenseite der Fußprothese (1) hin gerichtet ist.

6. Die Fußprothese (1) gemäß einem oder mehreren der obigen Ansprüche, **dadurch gekennzeichnet, dass** der vordere Abschnitt (3a) des ersten Auflagekörpers (3) sich näher an der Spitze (1a) der Fußprothese (1) befindet als der vordere Abschnitt (4a) des zweiten Auflagekörpers (4).

7. Die Fußprothese (1) gemäß einem oder mehreren der obigen Ansprüche, **dadurch gekennzeichnet, dass** sie Mittel (6) zur gegenseitigen Blockierung zwischen dem ersten Auflagekörper (3) und dem zweiten Auflagekörper (4) umfasst, die im Wesentlichen in den entsprechenden zweiten hinteren Bereichen angeordnet sind.

8. Die Fußprothese (1) gemäß einem oder mehreren der obigen Ansprüche, **dadurch gekennzeichnet, dass** der zweite Auflagekörper (4) den hinteren Auflageabschnitt (5) bestimmt.

9. Die Fußprothese (1) gemäß einem oder mehreren der obigen Ansprüche, **dadurch gekennzeichnet, dass** die hinteren Bereiche auf einer Anordnungsebene angeordnet sind, die im Verhältnis zur Anordnungsebene der vorderen Bereiche erhöht ist.

10. Die Fußprothese (1) gemäß einem oder mehreren der obigen Ansprüche, **dadurch gekennzeichnet, dass** sie mindestens einen plattenähnlichen Versteifungskörper (7) umfasst, der so angeordnet ist, dass er einem entsprechenden Verbindungsbereich zugewandt ist, der an dem ersten Auflagekörper (3) und/oder an dem zweiten Auflagekörper (4) bestimmt ist.

## Revendications

1. Pied artificiel (1) comprenant un élément d'appui (2) qui est destiné à reposer sur le sol et qui s'étend le long d'un axe d'extension longitudinal (100), ledit élément d'appui (2) étant associé à un corps d'accrochage (8) permettant sa fixation sur le corps de l'utilisateur, ledit élément d'appui (2) définissant une partie d'appui arrière (5) et deux parties d'appui avant (3a, 4a) qui sont mutuellement espacées transversalement par rapport à l'axe d'extension longitudinal (100) et qui sont adaptées pour recevoir des efforts, en utilisation, indépendamment l'une de l'autre,
ledit élément d'appui (2) comprend un premier corps d'appui (3) et un deuxième corps d'appui (4) qui ont une extension allongée,
**caractérisé en ce que** ledit premier corps d'appui (3) et ledit deuxième corps d'appui (4) ont une extension divergente par rapport à l'axe d'extension longitudinal (100), en allant de la partie d'appui arrière (5) vers les parties d'appui avant (3a, 4a), ledit premier corps d'appui (3) et ledit deuxième corps d'appui (4) définissant respectivement une région arrière et une région avant, au moins une partie (3b) de la région arrière dudit premier corps d'appui (3) étant agencée de façon à être en face d'au moins une partie (4b) de la région arrière dudit deuxième corps d'appui (4).

2. Pied artificiel (1) selon la revendication 1, **caractérisé en ce que** les régions avant respectives dudit premier corps d'appui (3) et dudit deuxième corps d'appui (4) sont disposées mutuellement côte à côte le long d'une direction qui est substantiellement transversale à l'axe d'extension longitudinal (100) dudit élément allongé (2).

3. Pied artificiel (1) selon l'une ou plusieurs des revendications précédentes, **caractérisé en ce que** la région arrière dudit premier corps d'appui (3) est placée au-dessus de la région arrière dudit deuxième corps d'appui (4).

4. Pied artificiel (1) selon l'une ou plusieurs des revendications précédentes, **caractérisé en ce que** la région avant dudit premier corps d'appui (3) a une plus grande extension dans une direction transversale que la région avant dudit deuxième corps d'appui (4).

5. Pied artificiel (1) selon l'une ou plusieurs des revendications précédentes, **caractérisé en ce que** la région avant dudit premier corps d'appui (3) est dirigée vers l'intérieur dudit pied artificiel (1) et **en ce que** la région avant dudit deuxième corps d'appui (4) est dirigée vers l'extérieur dudit pied artificiel (1).

6. Pied artificiel (1) selon l'une ou plusieurs des revendications précédentes, **caractérisé en ce que** la partie avant (3a) dudit premier corps d'appui (3) est placée plus près de l'orteil (1a) dudit pied artificiel (1) que la partie avant (4a) dudit deuxième corps d'appui (4).

7. Pied artificiel (1) selon l'une ou plusieurs des revendications précédentes, **caractérisé en ce qu'**il comprend des moyens (6) permettant un blocage mutuel entre ledit premier corps d'appui (3) et ledit deuxième corps d'appui (4) qui sont placés substantiellement au niveau des deuxièmes régions arrière respectives.

8. Pied artificiel (1) selon l'une ou plusieurs des revendications précédentes, **caractérisé en ce que** ledit deuxième corps d'appui (4) définit ladite partie d'appui arrière (5).

9. Pied artificiel (1) selon l'une ou plusieurs des revendications précédentes, **caractérisé en ce que** lesdites régions arrière sont disposées sur un plan de disposition qui est surélevé par rapport au plan de disposition desdites régions avant.

10. Pied artificiel (1) selon l'une ou plusieurs des revendications précédentes, **caractérisé en ce qu'**il comprend au moins un corps de renfort de forme plate (7) qui est agencé de façon à se trouver en face d'une région de connexion respective définie sur ledit premier corps d'appui (3) et/ou ledit deuxième corps d'appui (4).
